# EUROPÄISCHE PATENTSCHRIFT

(11) **EP 1 045 829 B1**
(45) Veröffentlichungstag und Bekanntmachung des Hinweises auf die Patenterteilung: **04.06.2003**
(21) Anmeldenummer: 98966672.2
(22) Anmeldetag: 24.12.1998
(51) Int. Cl.: C07C 231/02, C07C 233/43, C07C 235/56, C07C 269/04, C07C 271/28, C07C 303/38, C07C 311/08

(54) **VERFAHREN ZUR HERSTELLUNG VON N-(3-AMINO-4-FLUOR-PHENYL)-SULFONSÄUREAMIDEN, N-(3-AMINO-4-FLUOR-PHENYL)-CARBONSÄUREAMIDEN UND N-(3-AMINO-4-FLUOR-PHENYL)-CARBAMATEN**
METHOD FOR PRODUCING N-(3-AMINO-4-FLUOROPHENYL)- SULPHONAMIDES, N-(3-AMINO-4-FLUOROPHENYL) CARBOXYLIC ACID AMIDES AND N-(3-AMINO-4-FLUOROPHENYL) CARBAMATES
PROCEDE DE FABRICATION DE N-(3-AMINO-4-FLUORO-PHENYL) SULFONAMIDES, DE N-(3-AMINO-4-FLUORO-PHENYL) CARBONAMIDES ET DE N-(3-AMINO-4-FLUORO-PHENYL) CARBAMATES

(30) Priorität: 09.01.1998 DE 19800531
(43) Veröffentlichungstag der Anmeldung: 25.10.2000
(73) Patentinhaber: Bayer CropScience AG, 40789 Monheim (DE)
(72) Erfinder: HUPPERTS, Achim, D-40217 Düsseldorf (DE); LANTZSCH, Reinhard, D-42115 Wuppertal (DE)
(86) Internationale Anmeldenummer: EP9808443
(87) Internationale Veröffentlichungsnummer: WO99035122

(56) Entgegenhaltungen:
- EP-A- 0 496 595
- WO-A-97/27171
- DE-A- 2 725 146
- C.M. HALL, ET AL.: "Quinoline derivatives as antiallergy agents" JOURNAL OF MEDICINAL CHEMISTRY, Bd. 17, Nr. 7, Juli 1974, Seiten 685-690, XP002081502 Washington, DC, US
- I.W. HARVEY, ET AL.: "o-Nitroaniline derivatives. Part 11. 4- and 4-Amino-1H-benzimidazole-3-oxides" JOURNAL OF THE CHEMICAL SOCIETY, PERKIN TRANSACTIONS 1, Nr. 7, Juli 1988, Seiten 1939-1943, XP002101102 LETCHWORTH, GB
- C. YAMAGAMI, ET AL.: "Electronic properties of anticonvulsant amides. A C-13 nuclear magnetic resonance study of phenylacetanilides" CHEMICAL AND PHARMACEUTICAL BULLETIN, Bd. 31, Nr. 11, November 1983, Seiten 4172-4177, XP002101103 TOKYO, JP
- J.B. WRIGHT, ET AL.: "N,N'-(Phenylene)dioxamic acids and their esters as antiallergy agents" JOURNAL OF MEDICINAL CHEMISTRY, Bd. 21, Nr. 9, September 1978, Seiten 930-935, XP002101108 Washington, DC, US

## Beschreibung

Die Erfindung betrifft ein neues Verfahren zur Herstellung von N-(3-Amino-4-fluorphenyl)-sulfonsäureamiden, N-(3-Amino-4-fluor-phenyl)-carbonsäureamiden und N-(3-Amino-4-fluor-phenyl)-carbamaten, welche als Zwischenprodukte zur Herstellung von herbizid wirksamen Verbindungen verwendet werden können.

Es ist bekannt, daß man N-(3-Amino-4-fluor-phenyl)-sulfonsäureamide erhält, wenn man entsprechende N-(3-Nitro-4-fluor-phenyl)-sulfonsäureamide mit Reduktionsoder Hydrierungsmitteln, wie z.B. Eisen in Gegenwart einer Säure, wie z.B. Essigsäure, oder Wasserstoff in Gegenwart eines Katalysators, wie z.B. Platinoxid, umsetzt (vgl. EP-A-496595). Die Herstellung der hierbei benötigten Ausgangsstoffe durch selektive Reduktion von 1-Fluor-2,4-dinitro-benzol und anschließende Umsetzung mit Sulfonsäurechloriden ist jedoch technisch problematisch. Insbesondere die Versuche, 1-Fluor-2,4-dinitro-benzol selektiv zu 1-Fluor-4-amino-2-nitro-benzol zu hydrieren, führen stets zu Gemischen aus den möglichen einfach hydrierten Produkten und dem doppelt hydrierten Produkt. 1-Fluor-4-amino-2-nitro-benzol (vgl. EP-A-127079; Recueil Trav. Chim. Pays-Bas 65 (1946), 329) (und auch 1-Fluor-2,4-diamino-benzol (vgl. Bull. Soc. Chim. Fr. 132 (1995), 306-313)) werden daher im allgemeinen durch Reduktion mit Metallen oder mit Metallverbindungen, wie z.B. mit Eisen in Gegenwart von Eisen(II)-sulfat oder in Gegenwart von Essigsäure, oder mit Zinn(II)-chlorid in Gegenwart von Salzsäure, hergestellt. Die Verwendung solcher Reduktionsmittel sollte jedoch im industriellen Bereich wegen der damit verbundenen Entsorgungsprobleme möglichst vermieden werden.

Weiter ist bekannt, daß man N-(3-Amino-4-chlor-phenyl)-sulfonsäureamide oder N-(3-Amino-4-chlor-phenyl)-carbonsäureamide erhält, wenn man 1-Chlor-2,4-diaminobenzol mit Sulfonsäurechloriden bzw. mit Carbonsäurechloriden umsetzt (vgl. WO-A-9727171). Die Ausbeuten und die Qualitäten der Produkte sind bei diesen Umsetzungen jedoch nicht immer ganz zufriedenstellend. Da Chlor und Fluor als Substituenten an Arenen oft sehr unterschiedliche dirigierende Wirkung für weitere Reaktionen haben, konnte eine analoge Umsetzung von 1-Fluor-2,4-diamino-benzol mit Sulfonsäurechloriden oder Carbonsäurechloriden zudem auch nicht als selbstverständlich erwartet werden.

Es wurde nun gefunden, daß man N-(3-Amino-4-fluor-phenyl)-sulfonamide, N-(3-Amino-4-fluor-phenyl)-carbonsäureamide und N-(3-Amino-4-Fluor-phenyl)-carbamate der allgemeinen Formel (I) 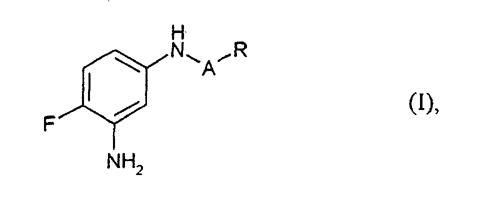 in welcher
- A: für SO₂, CO oder CO₂ steht und
- R: für jeweils gegebenenfalls substituiertes Alkyl oder Aryl steht,
in sehr guten Ausbeuten und in hoher Reinheit erhält, wenn man
1-Fluor-2,4-diamino-benzol der Formel (II) 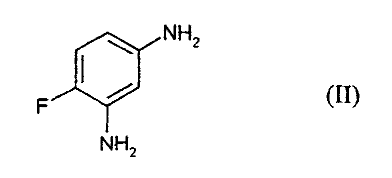 - oder Säureaddukte hiervon -
mit Sulfonsäurechloriden, Carbonsäurechloriden oder Chlorameisensäureestern der allgemeinen Formel (III) 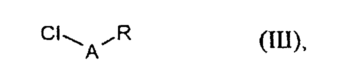 in welcher
- A und R: die oben angegebene Bedeutung haben,
in Gegenwart eines Säureakzeptors und und Gegenwart eines Verdünnungsmittels bei Temperaturen zwischen -20°C und +100°C umsetzt.

Überraschenderweise können nach dem erfindungsgemäßen Verfahren N-(3-Amino-4-fluor-phenyl)-sulfonsäureamide, N-(3-Amino-4-fluor-phenyl)-carbonsäureamide und N-(3-Amino-4-fluor-phenyl)-carbamate der allgemeinen Formel (I) in sehr guten Ausbeuten und in hoher Reinheit erhalten werden, obwohl das Anfallen angenähert äquimolarer Gemische der möglichen "einfachen" Sulfonylierungsprodukte bzw. Acylierungsprodukte - gegebenenfalls auch in größerem Umfang durch Produkte mehrfacher Sulfonylierung bzw. Acylierung verunreinigt - zu erwarten war.

Als Vorteil des erfindungsgemäßen Verfahrens sei erwähnt, daß das Ausgangsprodukt - 1 -Fluor-2,4-diamino-benzol - sehr leicht durch katalytische Hydrierung in einem Schritt aus 1-Fluor-2,4-dinitro-benzol erhalten werden kann und somit im Vergleich mit dem Stand der Technik (vgl. EP-A-496595) eine weitere Reduktionsstufe nicht mehr erforderlich ist.

Ein weiterer Vorteil des erfindungsgemäßen Verfahrens besteht darin, daß die Umsetzung nicht in einem Gemisch verschiedener Lösungsmittel (vgl. WO-A-9727171) durchgeführt werden muß, sondern ein Lösungsmittel genügt.

Besonders vorteilhaft ist zudem, daß die Umsetzungsprodukte der allgemeinen Formel (I) in vielen Fällen auch ohne Isolierung, d.h. im Reaktionsgemisch der erfindungsgemäßen Umsetzung, direkt für weitere Umsetzungen, wie z.B. Acylierungen oder Sulfonylierungen (mit Carbonsäurechloriden, Chlorameisensäureestern, Sulfonsäurechloriden etc.), eingesetzt werden können, welche in guten Ausbeuten zu reinen Produkten führen.

Das erfindungsgemäße Verfahren stellt somit eine wertvolle Bereicherung des Standes der Technik dar.

Verwendet man beispielsweise 1-Fluor-2,4-diamino-benzol und Methansulfonsäurechlorid als Ausgangsstoffe, so kann der Reaktionsablauf beim erfindungsgemäßen Verfahren durch das folgende Formelschema skizziert werden:

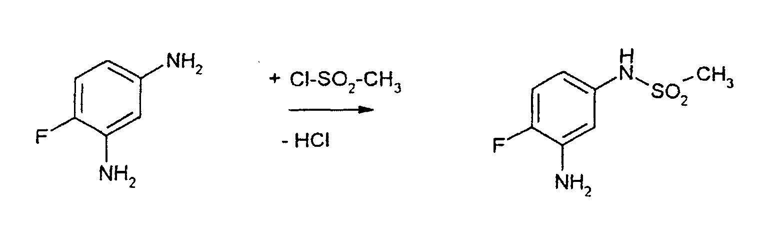

Die beim erfindungsgemäßen Verfahren als Ausgangsstoff zu verwendende Verbindung 1-Fluor-2,4-diamino-benzol der Formel (II) ist bereits bekannt (vgl. Bull. Soc. Chim. Fr. 132(1995), 306-313).

Die Verbindung der Formel (II) kann vorteilhaft durch katalytische Hydrierung von 1-Fluor-2,4-dinitro-benzol hergestellt werden.

Als Säureaddukte der Verbindung der Formel (II) können insbesondere Salze mit starken Säuren, wie Salzsäure oder Schwefelsäure eingesetzt werden. Als bevorzugt seien die Mono- und Bis-Hydrochloride genannt.
Die beim erfindungsgemäßen weiter als Ausgangsstoffe zu verwendenden Sulfonsäurechloride, Carbonsäurechloride und Chlorameisensäureester sind durch die Formel (III) allgemein definiert. In der Formel (III) stehen vorzugsweise
- A: für SO₂, CO oder CO₂ und
- R: für gegebenenfalls durch Cyano, Halogen oder C₁-C₄-Alkoxy substituiertes Alkyl mit bis 6 Kohlenstoffatomen oder für gegebenenfalls durch Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, C₁-C₄-Alkoxy-carbonyl oder Di-(C₁-C₄-alkyl)-amino substituiertes Aryl mit 6 oder 10 Kohlenstoffatomen.

In der Formel (III) stehen insbesondere
- A: für SO₂ oder CO und
- R: für jeweils gegebenenfalls durch Cyano, Fluor, Chlor, Methoxy oder Ethoxy substituiertes Methyl, Ethyl, n- oder i-Propyl, n-, i-, s- oder t-Butyl, oder für gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n-oder i-Propoxy-carbonyl, Dimethylamino oder Diethylamino substituiertes Phenyl.

Die Ausgangsstoffe der allgemeinen Formel (III) sind bekannte organische Synthesechemikalien.

Das erfindungsgemäße Verfahren wird unter Verwendung eines Säureakzeptors durchgeführt. Es kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kaliumoder Calcium-carbonat, Natrium-, Kalium- oder Calcium-hydrogencarbonat, Lithium-, Natrium-, Kalium- oder Calcium-hydrid, Lithium-, Natrium-, Kalium- oder Calciumhydroxid, Natrium- oder Kalium- -methanolat, -ethanolat, -n- oder -i-propanolat, -n-, - i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Ganz besonders werden basische organische Stickstoffverbindungen, insbesondere Pyridin, als Säureakzeptoren beim erfindungsgemäßen Verfahren bevorzugt.

Das erfindungsgemäße Verfahren wird unter Verwendung eines Verdünnungsmittels durchgeführt. Als Verdünnungsmittel zur Durchführung des erfindungsgemäßen Verfahrens kommen - gegebenenfalls neben Wasser - vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder - diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, gegebenenfalls auch (einoder mehrphasige) Gemische oben genannter Lösungsmittel mit Wasser.

Ganz besonders werden aprotisch polare organische Lösungsmittel, wie insbesondere Aceton, Butanon oder Methyl-isobutyl-keton; Acetonitril, Propionitril oder Butyronitril als Verdünnungsmittel beim erfindungsgemäßen Verfahren bevorzugt.

Die Reaktionstemperaturen können bei der Durchführung des erfindungsgemäßen Verfahrens in einem größeren Bereich variiert werden. Im allgemeinen arbeitet man bei Temperaturen zwischen -20°C und +100°C, vorzugsweise zwischen -5°C und +60°C, insbesondere zwischen +10°C und +30°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 100 bar - durchzuführen.

Zur Durchführung des erfindungsgemäßen Verfahrens setzt man pro Mol an 1-Fluor-2,4-diamino- benzol der Formel (II) im allgemeinen 0,9 bis 1,5 Mol, vorzugsweise 1,0 bis 1,1 Mol an Sulfonsäurechlorid bzw. Carbonsäurechlorid der Formel (III) und gegebenenfalls 1,0 bis 2,0 Moläquivalente, vorzugsweise 1,1 bis 1,5 Moläquivalente Säureakzeptor ein.

In einer bevorzugten Ausführungsform des erfindungsgemäßen Verfahrens wird das 1-Fluor-2,4-diamino-benzol der Formel (II) bei Raumtemperatur (ca. 20°C) mit einem Verdünnungsmittel und einem Säureakzeptor vermischt und unter Rühren wird dann das Sulfonsäurechlorid, das Carbonsäurechlorid oder der Chlorameisensäureester eindosiert. Alternativ können auch Säureakzeptor und Sulfonsäurechlorid, Carbonsäurechlorid oder Chlorameisensäureester zeitgleich ("synchron", "parallel") eindosiert werden. Die Reaktionsmischung wird bis zum Ende der Umsetzung im angegebenen Temperaturbereich gerührt und kann dann nach üblichen Methoden aufgearbeitet werden (vgl. die Herstellungsbeispiele).

Es ist aber auch möglich, die im Reaktionsgemisch vorliegenden Produkte der allgemeinen Formel (I) ohne Zwischenisolierung weiter mit Sulfonsäurechloriden, Carbonsäurechloriden oder Chlorameisensäureestern zu Verbindungen der allgemeinen Formel (IV) umzusetzen (vgl. die Herstellungsbeispiele) 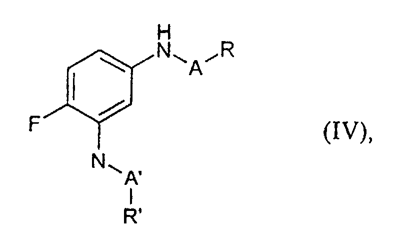 in welcher
- A und R: die oben angegebene Bedeutung haben und
- A' und R': die oben für A bzw. R angegebenen Bedeutungen haben, wobei jedoch die Bedeutungen von A und A' sowie R und R' nicht in jedem Einzelfall identisch sein müssen.

Die nach dem erfindungsgemäßen Verfahren herzustellenden Verbindungen der allgemeinen Formel (I) können als Zwischenprodukte zur Herstellung von herbizid wirksamen Verbindungen verwendet werden (vgl. EP-A-496595).

### Herstellungsbeispiele:

### Beispiel 1

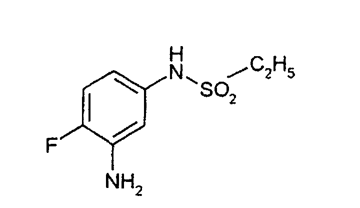

In einem 250ml Zweihalskolben mit Innenthermometer, Tropftrichter und Magnetrührer werden 6.30 g (0.05 Mol) 1-Fluor-2,4-diamino-benzol in 80 ml Acetonitril und 4.8 g (0.06 Mol) Pyridin vorgelegt und bei 10°C bis 15°C mit 6.43 g (0.05 Mol) Ethansulfonsäurechlorid versetzt. Die Reaktionsmischung wird eine Stunde bei Raumtemperatur (ca. 20°C) gerührt und mit 100 ml Essigsäureethylester und 50 ml Wasser versetzt. Die organische Phase wird abgetrennt, die wäßrige Lösung dreimal mit je 50 ml Essigsäureethylester extrahiert, die organischen Phasen vereinigt, mit 100 ml Wasser gewaschen, über Natriumsulfat getrocknet und filtriert. Vom Filtrat wird im Wasserstrahlvakuum das Lösungsmittel sorgfältig abdestilliert.

Man erhält 10.7 g Rohprodukt, das laut GC/MS 92.8% N-(3-Amino-4-fluor-phenyl)-ethansulfonsäureamid (d.h. 91% der Theorie) enthält.

### Beispiel 2

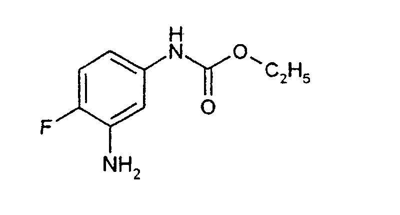

In einem 100ml Zweihalskolben mit Innenthermometer, Tropftrichter und Magnetrührer werden 3.15 g (25 mMol) 1-Fluro-2,4-diamino-benzol in 50 ml Acetonitril und 2.4 g (30 mMol) Pyridin vorgelegt und bei 10°C bis 15°C mit 2.7 g (25 mMol) Chlorameisensäureethylester versetzt. Die Reaktionsmischung wird eine Stunde bei Raumtemperatur (ca. 20°C) gerührt und dann mit 50 ml Methylenchlorid, 50 ml Wasser und 10 ml 2N-Salzsäure versetzt. Die organische Phase wird abgetrennt, die wässrige Lösung neutralisiert und dreimal mit je 20 ml Methylenchlorid extrahiert. Die vereinigten organischen Phasen werden mit 100 ml Wasser gewaschen, über Natriumsulfat getrocknet und filtriert. Vom Filtrat wird das Lösungsmittel im Wasserstrahlvakuum sorgfältig abdestilliert.

Man erhält 5.4 g Rohprodukt, das laut HPLC 91.8% N-(3-Amino-4-fluor-phenyl)-Oethyl-carbamat (100% der Theorie) enthält.

### Beispiel 3

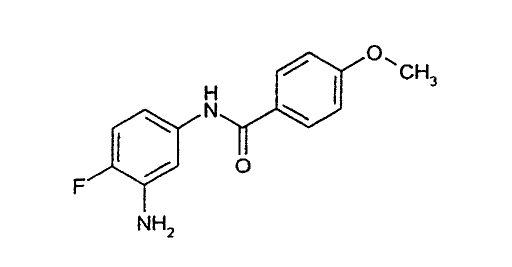

In einem 100 ml Zweihalskolben mit Innenthermometer, Tropftrichter und Magnetrührer werden 3.15 g (25 mMol) 1-Fluor-2,4-diamino-benzol in 50 ml Acetonitril und 2.4 g (30 mMol) Pyridin vorgelegt und bei 10°C bis 15°C mit 4.26 g (25 mMol) 4-Methoxy-benzoylchlorid versetzt. Die Reaktionsmischung wird eine Stunde bei Raumtemperatur (ca. 20°C) gerührt und dann mit 50 ml Essigsäureethylester und 50 ml Wasser versetzt. Die organische Phase wird abgetrennt, die wässrige Phase dreimal mit je 20 ml Essigsäureethylester nachextrahiert, die vereinigten organischen Phasen mit 100 ml Wasser gewaschen, über Natriumsulfat getrocknet und filtriert. Vom Filtrat wird im Wasserstrahlvakuum das Lösungsmittel sorgfältig abdestilliert.

Man erhält 6.1 g eines grauen kristallinen Rohproduktes, das laut HPLC 89.2% N-(3-Amino-4-fluor-phenyl)-4-methoxy-benzoesäureamid (84% der Theorie) enthält.

### Beispiel 4

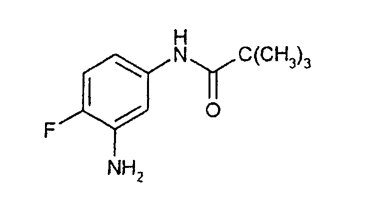

In einem 100ml Zweihalskolben mit Innenthermometer, Tropftrichter und Magnetrührer werden 3.15 g (25 mMol) 1-Fluor-2,4-diamino-benzol in 80 ml Acetonitril und 2.4 g (30 mMol) Pyridin vorgelegt und bei 5°C mit 3.01 g (25 mMol) Pivaloylchlorid versetzt. Die Reaktionsmischung wird eine Stunde bei Raumtemperatur (ca. 20°C) gerührt und mit 50 ml Essigsäureethylester und 50 ml Wasser versetzt. Die organische Phase wird abgetrennt, die wässrige Phase dreimal mit je 20 ml Essigsäureethylester nachextrahiert, die vereinigten organischen Phasen, mit 100 ml Wasser gewaschen, über Natriumsulfat getrocknet und filtriert. Vom Filtrat wird im Wasserstrahlvakuum das Lösungsmittel sorgfältig abdestilliert.

Man erhält 5.2 g eines braunen Feststoffes, der laut HPLC 82.7% N-(3-Amino-4-fluor-phenyl)-pivalinsäureamid (82% der Theorie) enthält.

### Beispiel 5

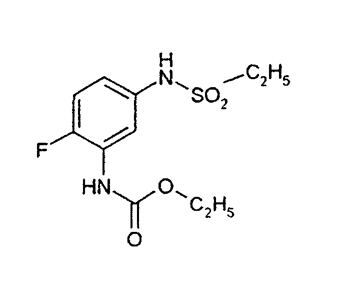

In einem 250ml Zweihalskolben mit Innenthermometer, Tropftrichter und Magnetrührer werden 6.30 g (50 mMol) 1-Fluor-2,4-diamino-benzol in 80 ml Acetonitril und 11.8 g (150 mMol) Pyridin vorgelegt und bei 10°C bis 15°C mit 6.43 g (50 mMol) Ethansulfonsäurechlorid versetzt. Die Reaktionsmischung wird eine Stunde bei Raumtemperatur (ca. 20°C) gerührt und dann mit 5.97 g (55 mMol) Chlorameisensäureethylester versetzt. Man rührt eine Stunde bei Raumtemperatur nach, versetzt das Reaktionsgemisch mit 100 ml Essigsäureethylester und 50 ml Wasser und trennt die Phasen. Die wässrige Phase wird dreimal mit je 50 ml Essigsäureethylester extrahiert, die vereinigten organischen Phasen mit 100 ml Wasser gewaschen, über Natriumsulfat getrocknet und filtriert. Vom Filtrat wird das Lösungsmittel im Wasserstrahlvakuum sorgfältig abdestilliert.

Man erhält 15.6 g eines kristallinen Rohproduktes, das laut HPLC 89.1% N-(3-Ethoxycarbonylamino-4-Fluor-phenyl)-ethansulfonsäureamid (96% der Theorie) enthält.

## Patentansprüche

1. Verfahren zum Herstellen von N-(3-Amino-4-fluor-phenyl)-sulfonamiden, N-(3-Amino-4-fluor-phenyl)-carbonsäureamiden oder N-(3-Amino-4-fluor-phenyl)-carbamaten der allgemeinen Formel (I) 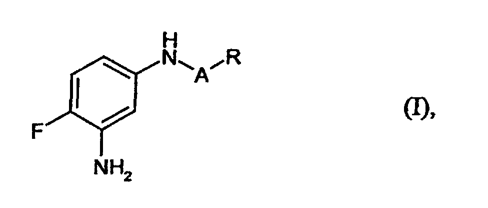 in welcher
A für SO₂, CO oder CO₂ steht und
R für jeweils gegebenenfalls substituiertes Alkyl oder Aryl steht,
**dadurch gekennzeichnet, dass** man
1-Fluor-2,4-diamino-benzol der Formel (II) 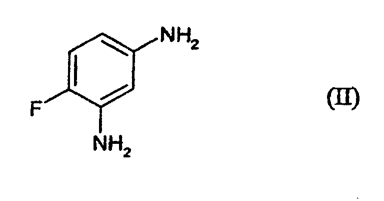 - oder Säureaddukte hiervon -
mit Sulfonsäurechloriden, Carbonsäurechloriden oder Chlorameisensäureestern der allgemeinen Formel (III) 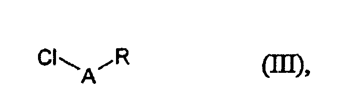 in welcher
A und R die oben angegebene Bedeutung haben,
in Gegenwart eines Säureakzeptors und in Gegenwart eines Verdünnungsmittels bei Temperaturen zwischen -20°C und +100°C umsetzt

2. Verfahren gemäß Anspruch 1, **dadurch gekennzeichnet, dass** man die Produkte der allgemeinen Formel (I) ohne Zwischenisolierung weiter mit Sulfonsäurechloriden, Carbonsäurechloriden oder Chlorameisensäureestern zu Verbindungen der allgemeinen Formel (IV) umsetzt, 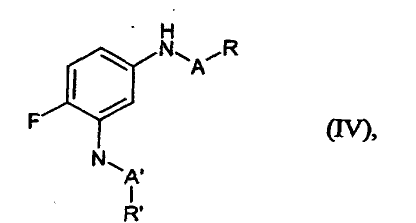 in welcher
A und R die in Anspruch 1 angegebene Bedeutung haben und
A' und R' die in Anspruch 1 für A bzw. R angegebenen Bedeutungen haben, wobei jedoch die Bedeutungen von A und A' sowie R und R' nicht in jedem Einzelfall identisch sein müssen.

3. Verfahren gemäß Anspruch 1, **dadurch gekennzeichnet, dass**
A für SO₂, CO oder CO₂ steht und
R für gegebenenfalls durch Cyano, Halogen oder C₁-C₄-Alkoxy substituiertes Alkyl mit 1 bis 6 Kohlenstoffatomen oder für gegebenenfalls durch Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, C₁-C₄-Alkoxy-carbonyl oder Di-(C₁-C₄-alkyl)-amino substituiertes Aryl mit 6 oder 10 Kohlenstoffatomen steht.

4. Verfahren gemäß Anspruch 1, **dadurch gekennzeichnet, dass**
A für SO₂ oder CO steht und
R für jeweils gegebenenfalls durch Cyano, Fluor, Chlor, Methoxy oder Ethoxy substituiertes Methyl, Ethyl, n- oder i-Propyl, n-, i-, s- oder t-Butyl, oder für gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n- oder i-Propoxy-carbonyl, Dimethylamino oder Diethylamino substituiertes Phenyl steht.

5. Verfahren gemäß einem der Ansprüche 1 bis 4, **dadurch gekennzeichnet, dass** nur ein einziges Verdünnungsmittel eingesetzt wird.

6. Verfahren gemäß einem der Ansprüche 1 bis 5, **dadurch gekennzeichnet, dass** als Säureakzeptor Pyridin eingesetzt wird.

7. Verfahren gemäß einem der Ansprüche 1 bis 6, **dadurch gekennzeichnet, dass** pro mol 1-Fluor-2,4-diamino-benzol der Formel (II) 0,9 bis 1,5 Mol Sulfonsäurechlorid bzw. Carbonsäurechlorid der Formel (III) und 1,0 bis 2,0 Moläquivalente Säureakzeptor eingesetzt werden.

8. Verfahren gemäß einem der Ansprüche 1 bis 7, **dadurch gekennzeichnet, dass** 1-Fluor-2,4-diamino-benzol der Formel (II) bei Raumtemperatur mit einem Verdünnungsmittel und einem Säureakzeptor vermischt und unter Rühren dann das Sulfonsäurechlorid, das Carbonsäurechlorid oder der Chlorameisensäureester eindosiert wird.

## Claims

1. Process for preparing N-(3-amino-4-fluoro-phenyl)-sulphonamides, N-(3-amino-4-fluoro-phenyl)-carboxamides or N-(3-amino-4-fluoro-phenyl)-carbamates of the general formula (I) 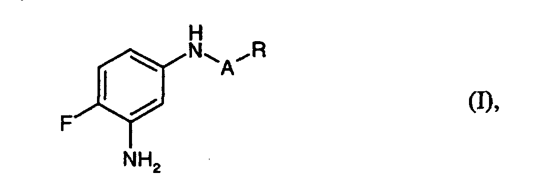 in which
A represents SO₂, CO or CO₂ and
R represents in each case optionally substituted alkyl or aryl,
**characterized in that**
1-fluoro-2,4-diamino-benzene of the formula (II) 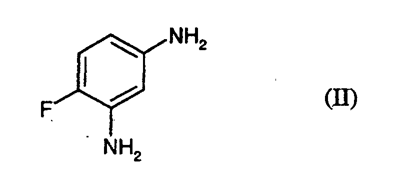 - or an acid adduct thereof -
is reacted with sulphonyl chlorides, carbonyl chlorides or chloroformic esters of the general formula (III) 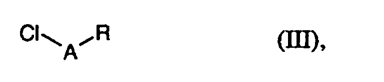 in which
A and R are as defined above
in the presence of an acid acceptor and in the presence of a diluent at temperatures between -20°C and +100°C.

2. Process according to Claim 1, **characterized in that** the products of the general formula (I) are reacted without intermediate isolation further with sulphonyl chlorides, carbonyl chlorides or chloroformic esters to give compounds of the general formula (IV) 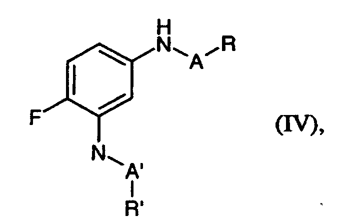 in which
A and R are as defined in Claim 1 and
A' and R' have the meanings given in Claim 1 for A and R, respectively, but where the meanings of A and A' and of R and R' do not have to be identical in each individual case.

3. Process according to Claim 1, **characterized in that**
A represents SO₂, CO or CO₂ and
R represents optionally cyano-, halogen- or C₁-C₄-alkoxy-substituted alkyl having 1 to 6 carbon atoms or represents optionally cyano-, nitro-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulphinyl-, C₁-C₄-halogenoalkylsulphinyl-, C₁-C₄-alkylsulphonyl-, C₁-C₄-halogenoalkylsulphonyl-, C₁-C₄-alkoxycarbonyl- or di-(C₁-C₄-alkyl)-amino-substituted aryl having 6 or 10 carbon atoms.

4. Process according to Claim 1, **characterized in that**
A represents SO₂ or CO and
R represents in each case optionally cyano-, fluorine-, chlorine-, methoxy- or ethoxy-substituted methyl, ethyl, n- or i-propyl, n-, i-, s- or t-butyl, or represents optionally cyano-, nitro-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methylthio-, ethylthio-, n- or i-propylthio-, trifluoromethylthio-, methylsulphinyl-, ethylsulphinyl-, n- or i-propylsulphinyl-, trifluoromethylsulphinyl-, methylsulphonyl-, ethylsulphonyl-, n- or i-propylsulphonyl-, trifluoromethylsulphonyl-, methoxycarbonyl-, ethoxycarbonyl-, n-or i-propoxy-carbonyl-, dimethylamino- or diethylamino-substituted phenyl.

5. Process according to any of Claims 1 to 4, **characterized in that** only one diluent is used.

6. Process according to any of Claims 1 to 5, **characterized in that** the acid acceptor used is pyridine.

7. Process according to any of Claims 1 to 6, **characterized in that** from 0.9 to 1.5 mol of sulphonyl chloride or carbonyl chloride of the formula (III) and from 1.0 to 2.0 molar equivalents of acid acceptor are employed per mole of 1-fluoro-2,4-diamino-benzene of the formula (II).

8. Process according to any of Claims 1 to 7, **characterized in that** 1-fluoro-2,4-diamino-benzene of the formula (II) is mixed at room temperature with a diluent and an acid acceptor and the sulphonyl chloride, the carbonyl chloride or the chloroformic ester is then metered in with stirring.

## Revendications

1. Procédé de production de N-(3-amino-4-fluorophényl) -sulfonamides, de N-(3-amino-4-fluorophényl)-carboxamides ou de N-(3-amino-4-fluorophényl)-carbamates de formule générale (I) 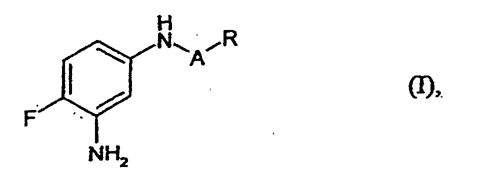 dans laquelle
A est un groupe SO₂, CO ou CO₂ et
R est un reste alkyle ou aryle dont chacun est éventuellement substitué,
**caractérisé en ce qu'**on fait réagir le 1-fluoro-2,4-diaminobenzène de formule (II) 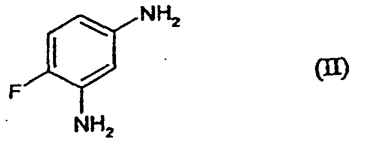 - ou des produits d'addition d'acides de ce composé -
avec des chlorures d'acide sulfonique, des chlorures d'acide carboxylique ou des esters d'acide chloroformique de formule générale (III) 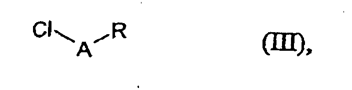 dans laquelle
A et R ont la définition indiquée ci-dessus,
en présence d'un accepteur d'acide et en présence d'un diluant, à des températures comprises entre -20°C et +100°C.

2. Procédé suivant la revendication 1, **caractérisé en ce qu'**on fait ensuite réagir les produits de formule générale (I) sans isolement intermédiaire avec des chlorures d'acide sulfonique, des chlorures d'acide carboxylique ou des esters d'acide chloroformique pour obtenir des composés de formule générale (IV) 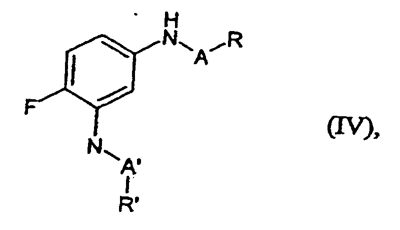 dans laquelle
A et R ont la définition indiquée dans la revendication 1 et
A' et R' ont les définitions indiquées pour A et pour R dans la revendication 1, les définitions de A et A' ainsi que de R et R' ne devant toutefois pas être identiques dans chaque cas individuel.

3. Procédé suivant la revendication 1, **caractérisé en ce que**
A est un groupe SO₂, CO ou CO₂ et
R est un reste alkyle ayant 1 à 6 atomes de carbone portant éventuellement un substituant cyano, halogéno ou alkoxy en C₁ à C₄, ou un reste aryle ayant 6 ou 10 atomes de carbone portant éventuellement un substituant cyano, nitro, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄, (alkoxy en C₁ à C₄)carbonyle ou di-(alkyle en C₁ à C₄)amino.

4. Procédé suivant la revendication 1, **caractérisé en ce que**
A est un groupe SO₂ ou CO et
R est un reste méthyle, éthyle, n-propyle, isopropyle, n-butyle, isobutyle, sec.-butyle ou tertio-butyle portant chacun le cas échéant, un substituant cyano,
fluoro, chloro, méthoxy ou éthoxy, ou un reste phényle portant éventuellement un substituant cyano, nitro, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, difluorométhoxy, trifluorométhoxy, méthylthio, éthylthio, n-propylthio, isopropylthio, trifluorométhylthio, méthylsulfinyle, éthylsulfinyle, n-propylsulfinyle, isopropylsulfinyle, trifluorométhylsulfinyle, méthylsulfonyle, éthylsulfonyle, n-propylsulfonyle, isopropylsulfonyle, trifluorométhylsulfonyle, méthoxycarbonyle, éthoxycarbonyle, n-propoxycarbonyle, isopropoxycarbonyle, diméthylamino ou diéthylamino.

5. Procédé suivant l'une des revendications 1 à 4, **caractérisé en ce qu'**un seul diluant est utilisé.

6. Procédé suivant l'une des revendications 1 à 5, **caractérisé en ce que** la pyridine est utilisée comme accepteur d'acide.

7. Procédé suivant l'une des revendications 1 à 6, **caractérisé en ce qu'**on utilise par mole de 1-fluoro-2,4-diaminobenzène de formule (II) 0,9 à 1,5 mole de chlorure d'acide sulfonique ou de chlorure d'acide carboxylique de formule (III) et 1,0 à 2,0 équivalents molaires d'accepteur d'acide.

8. Procédé suivant l'une des revendications 1 à 7, **caractérisé en ce qu'**on mélange le 1-fluoro-2,4-diamino-benzène de formule (II) à la température ambiante avec un diluant et un accepteur d'acide et on verse ensuite en agitant le chlorure d'acide sulfonique, le chlorure d'acide carboxylique ou l'ester d'acide chloroformique.
